# EUROPEAN PATENT SPECIFICATION

(11) **EP 0 294 529 B1**
(45) Date of publication and mention of the grant of the patent: **31.08.1994**
(21) Application number: 87305254.2
(22) Date of filing: 12.06.1987
(51) Int. Cl.: C12Q 1/04, G01N 1/34

(54) **Determination of bacteriuria**
Bestimmung von Bakteriuria
Détermination de la bactériurie

(43) Date of publication of application: 14.12.1988
(73) Proprietor: HYMAN, Edward S., New Orleans Louisiana 70125 (US)
(72) Inventor: HYMAN, Edward S., New Orleans Louisiana 70125 (US)
(74) Representative: Burford, Anthony Frederick

(56) References cited:
- WO-A-83/01779
- DE-A- 3 048 294
- FR-A- 2 436 993
- US-A- 4 379 135
- PATENT ABSTRACTS OF JAPAN, vol. 6, no. 27 (P-102)(905), 17 February 1982

## Description

This invention relates to a new method of detecting abnormal levels of bacteria in urine, and is relevant to the treatment of patients suffering from rheumatoid arthritis, essential hypertension, and other diseases in which significant bacteriuria was detected by the specimen preparation of the present invention that would not have been easily demonstrated by known procedures.

Under good conditions bacteria may be seen in an aqueous medium under the microscope at as low as 100 diameters magnification, but they are usually visualized at 1000 diameters magnification after drying and staining with appropriate dyes. Both methods of visualization have been used to examine urine. Much more commonly bacteria are demonstrated in urine by allowing them to grow in an appropriate culture medium until the colonies are visible to the naked eye. By counting the colonies and multiplying by the dilution of the urine, and by assuming that one colony represents a single bacterium or a cluster of bacteria in the original specimen, the number of bacteria in a cubic centimeter of urine may be estimated.

The direct microscopy and the culture methods each have pitfalls. In the past 20-25 years the direct visualization of bacteria in urine has largely been abandoned in favor of the methods involving culturing and counting the colonies of bacteria. Indeed virtually all of the studies of the significance of bacteriuria are based upon culturing the urine, and the direct microscopic examination of urine has been relegated to the status of a quick but inadequate screening procedure which may be helpful because it can be correlated with the culture methods.

Any culture method requires that the bacteria will grow in the laboratory in the medium selected and in the time allotted. If the bacteria are damaged or dead when they left the body, then they will not grow. There are many reasons why bacteria in urine would be damaged. The host's defence processes damage bacteria. The ionic strength or osmolality of the solution may be damaging. The oxidation potential of urine is usually too high (e.g., +.22 to +.25 volts). There may be a noxious metabolite in urine. (Human antibodies have been identified in urine and they have been demonstrated to be deposited on bacteria in urine.) Any or many of these factors may render a given bacterium non-viable in vitro. Finally, if the medium used is inappropriate for the growth of the particular organisms present, they will not grow.

It can be readily shown by staining and microscopy that many of the bacterial forms found under the microscope were not alive at the time the specimen was obtained. For example, some do not contain a nucleic acid, a biochemical component essential to life. Should all of the bacteria in a given specimen be devoid of nucleic acid, then none will grow and the culture of urine remains sterile. Indeed many urine specimens from sick patients containing huge numbers of bacteria will not yield a thriving bacterial culture in the hospital bacteriology laboratory. When the laboratory reports "no growth" the clinician may abandon the possibility of significant bacteriuria, and hence the possibility of an infectious cause. Nonetheless, these dead, damaged or fastidious bacteria, though they do not grow in culture, may in vivo have caused or exacerbated the patient's illness.

With regard to the direct examination of the urine, it must be noted that although bacteria may be seen in urine at only 100 diameters magnification, the size of the image is not the only consideration. Should the optical density and refractive index of a dead bacterium be near that of the medium, then it would not be detected by ordinary light microscopy. It may be seen by staining or perhaps by some specialized lighting (even then round bacteria cannot be distinguished from other near round particles such as crystals.) In the present method the urine is examined wet at 100 to 400 diameters magnification, but it is also dried and prepared in a particular way so as to retain and preserve the bacterial structure through staining. In particular, it has now been found that urine contains lipids which act as detergents. Should they be allowed to remain on the slide when an aqueous dye is applied to the slide, then much of the sediment (including bacteria) will wash off of the slide and the preparation will be lost. This is a major reason why past attempts to study bacteriuria have failed. Comparing one slide which had been prepared in the standard way with another which had been washed with a lipid solvent prior to chemically fixing, it is found that, after staining, much more sediment is found on the washed slide. Most of the sediment had washed off in the standard preparation. A chromatogram of the lipids removed reveals several lipids in the range of polarity of the phospholipids (e.g., lecithin, phosphatidylserine, etc. which substances act as detergents) but they do not contain appreciable phosphorus and thus they are not phospholipids. Standard methods of preparing and staining urine specimens, such as that of Melnick, US-A-4,225,669, do not provide for the precautionary removal of these lipids.

This invention utilizes a lipid wash to remove substances in urine which otherwise interfere with the retention of bacterial cells after aqueous staining. This is followed by chemical fixation which further fixes the specimen to the glass slide. By virtue of the improved specimen preparation method herein contemplated, it has been demonstrated that certain diseases of hitherto unknown or uncertain etiology are associated with bacteriuria not detected by prior methods. Application of antibiotic therapy appropriate to the detected organisms may then offer therapeutic benefit in the clinical illness. That improvement offers strong evidence of a causal relationship.

Those illnesses most frequently benefited by the use of antibiotic therapy following detection of bacteriuria by the present invention include: Rheumatoid Arthritis (and the related bursitis, tendonitis, tempero-mandibular arthritis, sacro-iliac arthritis, carpal-tunnel syndrome, temporal arteritis, palindromic rheumatism), and "essential" hypertension.

Although a limited number of cases have been investigated, diagnosed and successfully treated, it is believed that the present invention can also be relevant to the treatment of other diseases or conditions which include the following: rheumatic fever, systematic lupus erythematosis, scleroderma, classic migraine, transient ischemic attacks (TIAs) in the brain, mitral valve prolapse, urinary tract stones, reversible reduction in renal function, "brittle" diabetes mellitus, lymphangitis manifest as chronic brawny swelling or bacterial elephantiasis, otherwise unexplained edema, proteinuria, or fatigue, and many instances of diffuse backache.

The above disclosed technique for determining the presence of bacteria, especially cocci, in the urine - generally exceeding 20 cocci per cm³ - is useful as a general diagnostic technique which should be utilized in the diagnosis of the above listed diseases or conditions. Once the cocci have been found, an effective amount of an antibiotic effective against the cocci is administered.

According to the present invention, there is provided a method for direct microscopic detection of bacteria (including dead and damaged bacteria) in a urine sample including chemically fixing and staining the bacteria and microscopically observing the bacteria, characterised in that lipid components are removed from the sample by contact with a lipid solvent prior to fixing.

In the Drawings:

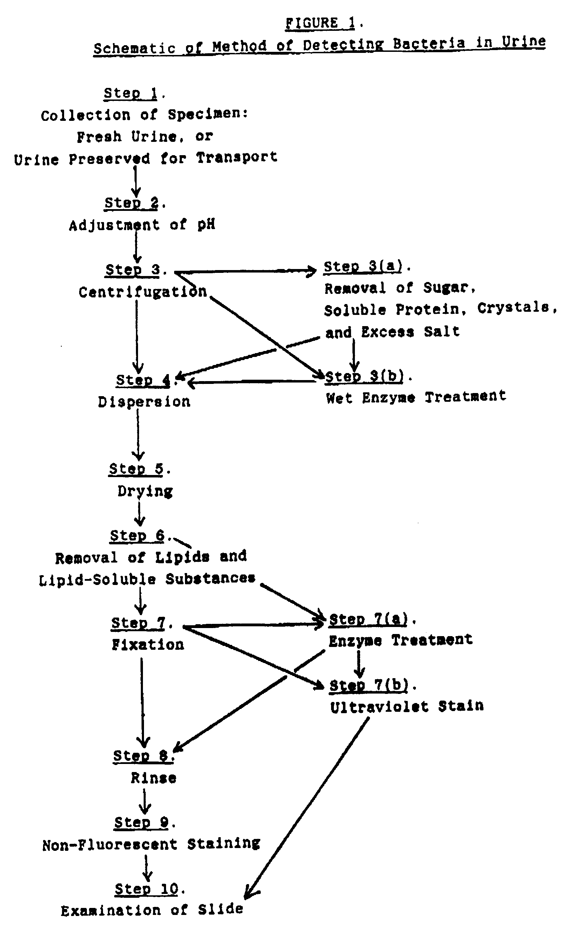

Fig 1 is a schematic diagram showing the steps of preferred embodiments of the method of the invention.

The examination and preparation steps of the presently preferred embodiment are described below:
1) Centrifugation: In the ordinary practice urine is centrifuged at a Relative Centrifugal Force (RCF) about 1000 times gravity or less. More commonly the RCF is not specified, is ambiguously stated, or is specified as so many revolutions per minute of a given centrifuge without specifying the radius of the rotor. Centrifugation of samples in the past has often been inadequate. Theoretically, a small particle may not sediment in any amount of time at too low a RCF (e.g. colloids). In practice, some bacteria will not sediment at the RCF of the "clinical centrifuge". Damaged bacteria may have a lower density approaching that of the urine, which itself varies in density from sample to sample. It is not infrequently important to apply a strong enough RCF to sediment all bacteria. Preferably, the urine is centrifuged at 4000 times gravity for 10-15 minutes. The tube may conveniently be conical, from 15 ml to 50 ml depending on the centrifuge.
2) Dispersion: After decanting the supernatant the sediment is dispersed in the remaining clear fluid (about 0.1 ml in an ordinary 15 ml conical tube) and the suspension is spread on a clean glass slide.
3) Wet viewing: The sediment is viewed without a coverslip at 100-400 diameters magnification. (A coverslip may damage casts and other formed elements, and removal of the coverslip would be necessary to stain the slide.)
4) Drying: The slide is then dried slowly, e.g., under the airstream of a low powered hair dryer.
5) Removal of Lipids: The lipids which have now been found in urine are in the range of polarity of the phospholipids. Preferably, they are washed off by a mixture of pure methanol and halogenated hydrocarbon (e.g., 1,1,1-trichlorethane) at 50:1.
6) Fixation: Although dilute glutaraldehyde in methanol is useful, if it is followed by a solution of copper phthalocyanine in methanol, the structures of cells and of damaged bacteria are better preserved.
7) Washing: preferably the slide is washed with pure methanol to remove residual copper phthalocyanine.
8) Staining: A conventional non-fluorescent stain, such as the Gram stain, may be used, as may a counterstain such as safranin. The slide is dried and examined at 1000 diameters without a coverslip.

Should the urine contain significant quantities of glucose (e.g., from a diabetic out of control) then the glucose will dry within the interstices of sediment on the slide and the sediment will not be fixed by the glutaraldehyde (GTL) or by the copper phthalocyanine (CuP) in methanol. This glucose will redissolve when an aqueous stain is applied , and as it redissolves will release the sediment. Similarly, when the urine contains 30 mg% or more of soluble protein (albumen, etc.), that soluble protein will interfere. Unlike glucose, that protein will be fixed by the GTL and CuP and will form a brittle film on the slide. Large portions of this film may break off in the staining procedure. Even then there is a remarkable tendency for the released film to leave behind the formed elements (especially the bacteria) which remain fixed to the slide. That portion of the homogeneous film of protein which remains on the slide stains much like the formed elements, and during the examination for bacteria using the oil objective (1000 x), the stained protein film may obscure bacteria and important formed elements in the sediment (white blood cells, red blood cells, tubular epithelial cells, and casts). Since the bacteria are usually demonstrable either inside the protein or in the areas of the slide free of protein film, the preparation is not completely lost.

In addition to the urines containing excessive amounts of glucose and of protein, a few urines contain other soluble non-lipid material (perhaps phosphates) which does not fix to the slide and which releases the sediment from the slide. All of these urines are better studied after washing the sediment. Often there is enough sediment remaining in the test tube used in the initial centrifuging to proceed with a wash.

Thus, in a modification of the method, in place of Step 2 above the sediment is washed with a solution, preferably ionic in nature, preferably lightly hypertonic to plasma (such as 0.2 molar NaCl), which solution has been passed through a 0.22 µm filter to render it bacteriologically sterile and particle free.

In order to wash the packed sediment of the fresh urine (of the usual preparation above) it is dispersed in about 3 ml of wash solution and centrifuged, e.g. 4000 g for 5 minutes. The supernatant solution is decanted and the sediment is washed with another 3 ml. The twice washed sediment is spread on a slide where under low magnification (100 x) formed elements (casts, white blood cells, red blood cells, tubular epithelial cells, bacteria) are more readily seen than washed sediment. When dried on the slide the washed sediment adheres very well to the glass through delipidation, fixation and staining. The gross appearance of Gram-stained slide is different (more dense, and red instead of blue) because some material (presumably glycoprotein) which stains blue with the copper phthalocyanine has been removed. However, the Gram stain is not altered. Gram positive bacteria still take the positive stain. But having removed most of the protein and glucose, Gram negative sediment (bacteria, cells, casts), previously obscured by the similar homogeneous stain of precipitated soluble protein, now stands out.

Since a heavy deposit of copper phthalocyanine will absorb ultraviolet light, this fixative cannot be used for UV fluorescence staining. Step # 6 may be modified to permit UV fluorescence staining by either increasing the glutaraldehyde concentration or the duration of exposure to the fixative. Then the slide may be stained with the UV fluorescing stain, e.g., a stabilized solution of acridine orange. Acridine orange, and most similar cationic dyes, themselves exert a fixative effect, and so use of the UV fluorescing stain may in some instances be substituted for use of a fixative.

In a further modification of the present method of specimen preparation, insoluble proteins are removed by bacterial or fungal proteases, or proteolytic enzymes of animal origin such as crystalline trypsin and chymotrypsin. The enzyme may be used after fixation of the sediment to the slide, but it is preferable to use it before the wash.

A slide prepared in any of the above procedures may be treated with a proteolytic enzyme after fixation with GTL, but preferably before fixation with CuP. A solution of enzyme in saline is simply applied to the slide. The slide is incubated at room temperature or at 37 degrees C, washed with saline, fixed again and stained.

It is preferable to treat the sediment with enzyme in the test tube before the fixative is added. Fresh urine is centrifuged at 4000 g for 10-15 minutes without fixative or preservative. The supernatant is decanted and crystalline trypsin (or a strong solution of bacterial protease) is added to the sediment. After stirring the tube is incubated (preferably at 37 degrees C) for 10 minutes and then centrifuged at 4000 g for 5 minutes. The drop of new supernatant is drained off, and the sediment is washed twice as above.

This treatment removes some of the insoluble proteins. Two advantages are noted. First, some of the sediment is removed but bacteria and even degenerate bacteria, cells, and casts are spared. This provides a means to concentrate important sediment such as bacteria. Second, the staining of some bacteria is changed. Most notable is the emergence of Gram positive cocci in sediments that contained only Gram negative cocci in their unwashed or washed preparations. Since the Gram positive material is peculiar to the cell wall of these bacteria, it is quite unlikely that each of two proteolytic enzymes would create the cell wall conditions for a positive stain (retention of the iodinated crystal violet). Instead it is quite likely that each proteolytic enzyme removed a protein, for example a human antibody adhering to the bacterial cell wall, that prevented the Gram positive dye from penetrating or fixing inside the cell wall.

In still other embodiments, the urine sediment is treated with other enzymes, or antibodies to reveal additional information. Among the enzymes that may be so employed are amylase (to remove carbohydrate polymers), DNases, RNases, lipases, lechithinases, sphingomyelinases, sialases, neuraminidases, and hyaluronidases. Among the dyes which may be employed is acridine orange, which may be used to demonstrate nucleic acids by fluorescence. Among the antibodies which may be utilized are tagged (e.g., fluorescent) anti-human IgG, polyclonal or monoclonal, to demonstrate the presence of the human IgG on the bacteria of the sediment.

By the method of the present invention, small cocci have been consistently found in the urine of patients with various forms of rheumatoid arthritis, systemic lupus erythematosis, and rheumatic fever and, in those instances examined, patients with migraine, bursitis, tendonitis, temporal arteritis, and diffuse persistent backache. It appears that a large array of seemingly unrelated illnesses may, by this approach, become reclassified as illnesses which are related to the appearance of small cocci in the urine, and that, from the beneficial effects of antibacterial treatment, such a reclassification may be meaningful. The actual diverse illnesses mentioned above may simply be responses to the same bacterial invasion or to similar bacterial invasions and the diversity (or different illnesses by current classification) may simply be varied responses by the human host to a given invasion.

Moreover, this novel preparation procedure has been used to detect the association of larger or damaged ("exploded") cocci in the urine of patients suffering from hypertension, transient ischemia attacks (TIA's) of the brain, and in the few cases seen, mitral valve prolapse and IgA nephropathy. Again these seemingly unrelated illnesses may simply be various responses to the invasion by said bacteria. Indeed most of these illnesses have in common an early lesion in the tiny arteries in the organs involved.

Numerous cases of Rheumatoid Arthritis (RA) examined have also had large numbers of small coccus in the urine. 26 cases of RA seen each had small cocci in the urine in great numbers, often hundreds of cocci per 1,000 x, oil immersion field, and each has had significant improvement of the illness on antibacterial therapy without any other change in medication. Some have even had a full remission of the illness with eradication of the bacteriuria.

## Claims

1. A method for direct microscopic detection of bacteria in a urine sample including chemically fixing and staining the bacteria and microscopically observing the bacteria, characterised in that lipid components are removed from the sample by contact with a lipid solvent prior to fixing.

2. A method as claimed in Claim 1, wherein the sample is centrifuged at at least 3500 times gravity to sediment bacteria in the sample prior to removal of lipid components.

3. A method as claimed in Claim 2, wherein the centrifuging is conducted at 3500 to 4000 times gravity.

4. A method as claimed in any one of the preceding claims, wherein the lipid solvent is methanol or a mixture of methanol and halogenated hydrocarbon.

5. A method as claimed in Claim 4, wherein the lipid solvent is a mixture of methanol and 1,1,1-trichloroethane.

6. A method as claimed in any one of the preceding claims, comprising:
a) centrifuging the sample at sufficient force and for sufficient time to sediment bacteria;
b) separating the sediment from the supernatant;
c) spreading the sediment on a surface;
d) drying the sediment;
e) washing the dried sediment on the surface with a lipid solvent to remove lipid components of the sediment;
f) chemically fixing the sediment;
g) staining the fixed sediment; and
h) microscopically observing the stained sediment.

7. A method as claimed in Claim 6, wherein the deposited sediment is wet viewed without a coverslip at 100 to 400 diameters magnification.

8. A method as claimed in Claim 6, wherein the deposited sediment is washed with an ionic solution, which solution has been passed through a filter to render it bacteriologically sterile and particle free.

9. A method as claimed in any one of Claims 6 to 8, wherein insoluble proteins are removed by incubation of the lipid solvent-washed sediment with bacterial or fungal protease or proteolytic enzyme before or after fixation.

10. A method as claimed in Claim 9, wherein the enzyme is crystalline trypsin or chymotrypsin.

11. A method as claimed in any one of Claims 6 to 10, wherein the sediment is contacted with a tagged anti-human IgG antibody to demonstrate the presence of human IgG on the bacteria of the sediment.

12. A method as claimed in Claim 11, wherein the antibody is tagged with a fluorescent dye.

13. A method as claimed in any one of Claims 6 to 10, wherein the lipid solvent-washed sediment is contacted with acridine orange to demonstrate nucleic acids by fluorescence.

## Patentansprüche

1. Verfahren zum direkten mikroskopischen Nachweis von Bakterien in einer Urinprobe, welches umfaßt chemisches Fixieren und Anfärben der Bakterien und mikroskopisches Beobachten der Bakterien, dadurch gekennzeichnet, daß die Lipidkomponenten vor dem Fixieren durch Kontakt mit einem Lipidlösungsmittel aus der Probe entfernt werden.

2. Verfahren nach Anspruch 1, worin die Probe mindestens mit 3500-facher Schwerkraft zentrifugiert wird, um die Bakteriein in der Probe vor dem Entfernen der Lipidkomponenten zu sedimentieren.

3. Verfahren nach Anspruch 2, worin das Zentrifugieren bei 3500- bis 4000-facher Schwerkraft durchgeführt wird.

4. Verfahren nach einem der vorangegangenen Ansprüche, worin das Lipidlösungsmittel Methanol oder ein Gemisch aus Methanol und halogenierten Kohlenwasserstoffen ist.

5. Verfahren nach Anspruch 4, worin das Lipidlösungsmittel ein Gemisch aus Methanol und 1,1,1-Trichlorethan ist.

6. Verfahren nach einem der vorangegangenen Ansprüche, welches umfaßt:
a) Zentrifugieren der Probe mit ausreichender Kraft und ausreichender Zeit, um die Bakterien zu sedimentieren,
b) Abtrennen des Sediments vom Überstand ,
c) Sprühen des Sediments auf eine Oberfläche,
d) Trocknen des Sediments,
e) Waschen des getrockneten Sediments auf der Oberfläche mit einem Lipidlösungsmittel, um die Lipidkomponenten aus dem Sediment zu entfernen,
f) chemisches Fixieren des Sediments,
g) Anfärben des fixierten Sediments, und
h) mikroskopisches Beobachten des angefärbten Sediments.

7. Verfahren nach Anspruch 6, worin das abgelagerte Sediment naß beobachtet wird ohne Abdeckverschiebung bei 100 bis 400 Durchmesservergrößerung.

8. Verfahren nach Anspruch 6, worin das abgelagerte Sediment mit einer ionischen Lösung gewaschen wird, wobei die Lösung durch einen Filter geleitet wurde, um diese bakteriologisch steril und teilchenfrei zu machen.

9. Verfahren nach einem der Ansprüche 6 bis 8, worin unlösliche Proteine durch Inkubieren des Lipidlösungsmittel/gewaschenes Sediment mit Protease aus Bakterien oder Pilzen oder proteolytischen Enzym vor oder nach dem Fixieren entfernt werden.

10. Verfahren nach Anspruch 9, worin das Enzym kristallines Trypsin oder Chymotrypsin ist.

11. Verfahren nach einem der Ansprüche 6 bis 10, worin das Sediment in Kontakt gebracht wird mit einem markierten anti-human IgG-Antikörper, um die Gegenwart von menschlichem IgG auf den Bakterien des Sediments zu zeigen.

12. Verfahren nach Anspruch 11, worin der Antikörper mit einem fluoreszierenden Farbstoff markiert wird.

13. Verfahren nach einem der Ansprüche 6 bis 10, worin das Lipidlösungsmittel/gewaschenes Sediment mit Acridin-Orange in Kontakt gebracht wird, um Nucleinsäuren durch Fluoreszenz zu zeigen.

## Revendications

1. Procédé de détection microscopique directe de bactéries dans un échantillon d'urine comprenant la fixation chimique et la coloration des bactéries et l'observation microscopique des bactéries, caractérisé en ce que les composants lipidiques sont enlevés de l'échantillon par contact avec un solvant des lipides avant la fixation.

2. Procédé selon la revendication 1, dans lequel l'échantillon est centrifugé à au moins 3500 g pour sédimenter les bactéries dans l'échantillon avant l'enlèvement des composants lipidiques.

3. Procédé selon la revendication 2, dans lequel la centrifugation est effectuée à 3500-4000 g.

4. Procédé selon l'une quelconque des revendications précédentes, dans lequel le solvant des lipides est le méthanol ou un mélange de méthanol et d'hydrocarbure halogéné.

5. Procédé selon la revendication 4, dans lequel le solvant des lipides est un mélange de méthanol et de 1,1,1-trichloroéthane.

6. Procédé selon l'une quelconque des revendications précédentes, consistant :
a) à centrifuger l'échantillon à une force suffisante et pendant un temps suffisant pour faire sédimenter les bactéries;
b) à séparer le sédiment du surnageant;
c) à étaler le sédiment sur une surface;
d) à sécher le sédiment;
e) à laver le sédiment séché sur la surface avec un solvant des lipides pour enlever les composants lipidiques du sédiment;
f) à fixer chimiquement le sédiment;
g) à colorer le sédiment fixé; et
h) à observer au microscope le sédiment coloré.

7. Procédé selon la revendication 6, dans lequel le sédiment déposé est humide vu sans lamelle à un grossissement des diamètres de 100 à 400.

8. Procédé selon la revendication 6, dans lequel le sédiment déposé est lavé avec une solution ionique, solution que l'on a fait passer à travers un filtre pour la rendre bactériologiquement stérile et exempte de particules.

9. Procédé selon l'une quelconque des revendications 6 à 8, dans lequel les protéines insolubles sont éliminées par incubation du sédiment lavé au solvant des lipides, à l'aide d'une protéase bactérienne ou fongique ou d'une enzyme protéolytique avant ou après fixation.

10. Procédé selon la revendication 9, dans lequel l'enzyme est la trypsine ou la chimotrypsine cristallines.

11. Procédé selon l'une quelconque des revendications 6 à 10, dans lequel le sédiment est mis en contact avec un anticorps anti-IgG humaine marqué pour démontrer la présence d'IgG humaine sur les bactéries du sédiment.

12. Procédé selon la revendication 11, dans lequel l'anticorps est marqué par un colorant fluorescent.

13. Procédé selon l'une quelconque des revendications 6 à 10, dans lequel le sédiment lavé au solvant des lipides est mis en contact avec de l'orangé d'acridine pour mettre en évidence les acides nucléiques par fluorescence.
